# EUROPEAN PATENT SPECIFICATION

(11) **EP 3 099 169 B1**
(45) Date of publication and mention of the grant of the patent: **25.11.2020**
(21) Application number: 15743919.1
(22) Date of filing: 29.01.2015
(51) Int. Cl.: A01N 37/40, C07D 213/79, A01N 43/40

(54) **METHODS FOR CONTROL OF AQUATIC WEEDS USING HERBICIDAL 4-AMINO-3-CHLORO-6-(4-CHLORO-2-FLUORO-3-METHOXYPHENYL)PYRIDINE-2-CARBOXYLIC ACIDS**
VERFAHREN ZUR KONTROLLE VON WASSERUNKRAUT MIT HERBIZIDEN 4-AMINO-3-CHLOR-6-(4-CHLOR-2-FLUOR-3-METHOXYPHENYL)PYRIDIN-2-CARBOXYLSÄUREN
PROCÉDÉS DE LUTTE CONTRE LES MAUVAISES HERBES AQUATIQUES À L'AIDE D'ACIDES 4-AMINO-3-CHLORO-6-(4-CHLORO-2-FLUORO-3-MÉTHOXYPHÉNYL)PYRIDINE-2-CARBOXYLIQUES HERBICIDES

(30) Priority: 31.01.2014 US 201461934007 P
(43) Date of publication of application: 07.12.2016
(73) Proprietor: Dow AgroSciences LLC, Indianapolis, IN 46268 (US)
(72) Inventor: MANN, Richard, K., Indianapolis, IN 4626846131 (US)
(74) Representative: f & e patent
(86) International application number: PCT/US2015/013396
(87) International publication number: WO 2015/116745

(56) References cited:
- WO-A2-2007/082098
- WO-A2-2009/029518
- US-A1- 2009 011 934
- US-A1- 2013 143 738
- US-A1- 2014 031 209
- Anonymous: "Alternanthera philoxeroides - Wikipedia", , 14 November 2019 (2019-11-14), XP055652782, Retrieved from the Internet: URL:https://en.wikipedia.org/wiki/Alternan thera_philoxeroides [retrieved on 2019-12-13]
- Anonymous: "Pond Weed Types - Aquatic Weed Identification Information", , 1 January 2019 (2019-01-01), XP055652779, Retrieved from the Internet: URL:https://www.lakerestoration.com/c-68-w eed-types.aspx [retrieved on 2019-12-13]
- Anonymous: "Heteranthera limosa - Wikipedia", , 23 March 2019 (2019-03-23), XP55652775, Retrieved from the Internet: URL:https://en.wikipedia.org/wiki/Heterant hera_limosa [retrieved on 2019-12-13]

## Description

### Background

Aquatic plants commonly arise as undesired weeds in waters and wetlands in the U.S. and other countries. For instance the publication "Integrated Pest Management for Rice" 2nd Ed., University of California, Statewide Integrated Pest Management Project, Publication 3280, 1993 illustrates common weeds, including aquatic weeds, in the different habitats of California rice fields. Three such exotic weeds are hydrilla, curlyleaf pondweed, and watermilfoil, including Eurasian watermilfoil, which are found in ponds, lakes, and other water bodies. The treatment of these bodies of water to eliminate or control the undesired or exotic aquatic weeds is often complicated by the fact that the agent used to control the undesired weed also adversely affects the health of desirable or native plant life. Aquatic herbicides need to be in contact with aquatic or submersed aquatic plants for a period of time (exposure time), which is dependent on the individual agent and the concentration at which it is used. Some herbicides require long exposures (one or more months) to control certain submersed, immersed or floating plants in water, which can adversely affect non-target species. Long exposure times can be difficult to achieve in a fluid environment. Insufficient exposure can lead to poor efficacy or failed treatments. Thus, methods or techniques to reduce exposure times and/or reduce the concentrations of agents used to control submersed weeds may benefit efficacy and/or selectivity.

The efficacy of herbicidal agents against the target aquatic weeds depends on several factors, including the application dose, the specific formulation, the plant type, climatic conditions, water and sediment conditions, herbicide exposure time, etc. At times, an inability to control an undesired weed can be overcome simply by increasing the rate of application or concentration for a particular herbicidal agent. However, this is not always the case, and higher rates of application can exacerbate adverse or undesired effects on beneficial plants and aquatic organisms and may not adequately compensate for insufficient contact with the targeted plant.

The use of quinoline monocarboxylic acids for controlling aquatic weeds, including submersed weeds, is disclosed in US 2009/0011934 A1.

WO 2009/029518 A2, US 2013/0143738 A1 and US 2014/0031209 A1 relate to herbicidal compositions comprising pyridine carboxylic acid derivatives.

### Summary

Described herein are methods for controlling aquatic weeds in a body of water. The methods include providing in the body of water a composition containing a herbicidally effective amount of a compound of formula I 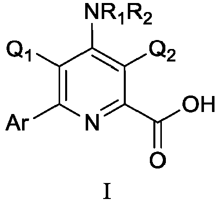 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group selected from the group consisting of
   a) 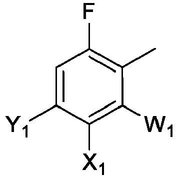 wherein
      W₁ represents halogen;
      X₁ represents F, Cl, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -CN, -NR₃R₄ or fluorinated acetyl or propionyl;
      Y₁ represents C₁-C₄ alkyl, C₁-C₄ haloalkyl, halogen or -CN, or, when X₁ and Y₁ are taken together, represents -O(CH₂)ₙO- wherein n = 1 or 2; and
      R₃ and R₄ independently represent H or C₁-C₄ alkyl;
   b) 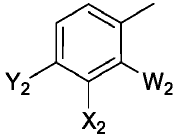 wherein
      W₂ represents F or Cl;
      X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
      Y₂ represents halogen, C₁-C₄ alkyl, C₁-C₄ haloalkyl or -CN, or, when W₂ represents F, X₂ and Y₂ taken together represent -O(CH2₎ₙO- wherein n = 1 or 2; and
      R₃ and R₄ independently represent H or C₁-C₆ alkyl; and
   c) 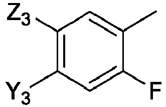 wherein
      Y₃ represents halogen, -CN or -CF₃;
      Z₃ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl; and
      R₃ and R₄ independently represent H, or C₁-C₆ alkyl;
   and agriculturally acceptable derivatives of the carboxylic acid group,
   wherein the aquatic weeds are selected from submersed aquatic plants.

Preferred compounds of formula I include 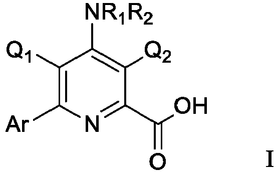 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group 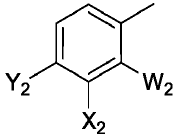 wherein
   W₂ represents F or Cl;
   X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
   Y₂ represents halogen, C₁-C₄ alkyl, C₁-C₄ haloalkyl or -CN, or, when W₂ represents F, X₂ and Y₂ taken together represent -O(CH₂) ₙO- wherein n = 1 or 2; and R₃ and R₄ independently represent H or C₁-C₆ alkyl;
   and agriculturally acceptable derivatives of the carboxylic acid group.

Particularly preferred compounds of formula I include 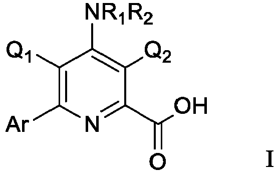 wherein
Q₁ represents H or F;
Q₂ represents Cl;
R₁ and R₂ represent H; and
Ar represents a polysubstituted aryl group 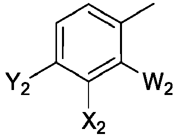 wherein
   W₂ represents F;
   X₂ represents C₁-C₄ alkoxy;
   Y₂ represents Cl;
   and agriculturally acceptable derivatives of the carboxylic acid group. In some embodiments, X₂ is methoxy.

In these methods, the compounds of formula I are unexpectedly effective when applied to aquatic plants at very low concentrations, and/or can be used to improve selectivity for target aquatic weeds, thus having less adverse effect on non-target plant species due to reduced concentration of, and/or exposure to, the herbicidal agents. In one embodiment, the final dilution level of the compound of formula I in the body of water is from about 5 parts-per-billion (ppb) to about 2000 ppb, preferably from about 5 parts-per-billion (ppb) to about 200 ppb.

In certain specific embodiments, aquatic weeds, for example hydrilla, curlyleaf pondweed, and watermilfoil, can be effectively controlled by compounds of formula I. Preferred compounds of formula I are methyl 4-amino-3-chloro-6-(4-chloro-2-fluoro-3-methoxyphenyl)pyridine-2-carboxylate (halauxifen-methyl) and benzyl 4-amino-3-chloro-5-fluoro-6-(4-chloro-2-fluoro-3-methoxyphenyl)pyridine-2-carboxylate.

In other embodiments, the methods further include the use of an additional pesticide, e.g., diquat dibromide, copper salts, endothal, flumioxazin, carfentrazone-ethyl, fluridone, topramezone, 2,4-D, triclopyr, penoxsulam, imazamox, bispyribac-sodium, or agriculturally acceptable salts or esters thereof.

### Detailed Description

### I. Definitions

Compounds of formula I belong to a family of compounds with a broad spectrum of weed control activity against woody plants, broadleaf, grass, and sedge weeds and are described in U.S. Patent 7,314,849 (B2), which is incorporated herein by reference in its entirety. As used herein, the compounds of formula I have the following structure: 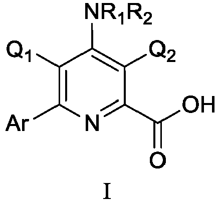 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group selected from the group consisting of
   a) 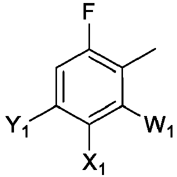 wherein
      W₁ represents halogen;
      X₁ represents F, Cl, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -CN, -NR₃R₄ or fluorinated acetyl or propionyl;
      Y₁ represents C₁-C₄ alkyl, C₁-C₄ haloalkyl, halogen or -CN, or, when X₁ and Y₁ are taken together, represents -O(CH₂)ₙO- wherein n = 1 or 2; and
      R₃ and R₄ independently represent H or C₁-C₄ alkyl;
   b) 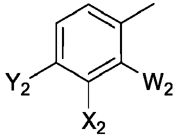 wherein
      W₂ represents F or Cl;
      X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
      Y₂ represents halogen, C₁-C₄ alkyl, C₁-C₄ haloalkyl or -CN, or, when W₂ represents F, X₂ and Y₂ taken together represent -O(CH₂)ₙO- wherein n = 1 or 2; and
      R₃ and R₄ independently represent H or C₁-C₆ alkyl; and
   c) 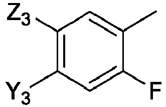 wherein
      Y₃ represents halogen, -CN or -CF₃;
      Z₃ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl; and
      R₃ and R₄ independently represent H, or C₁-C₆ alkyl;
   and agriculturally acceptable derivatives of the carboxylic acid group.

Preferred compounds of formula I include 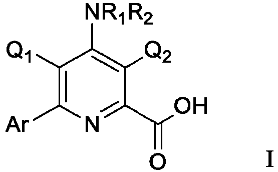 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group 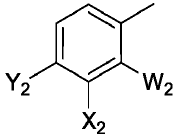 wherein
   W₂ represents F or Cl;
   X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
   Y₂ represents halogen, C₁-C₄ alkyl, C₁-C₄ haloalkyl or -CN, or, when W₂ represents F, X₂ and Y₂ taken together represent -O(CH₂)ₙO- wherein n = 1 or 2; and
   R₃ and R₄ independently represent H or C₁-C₆ alkyl;
   and agriculturally acceptable derivatives of the carboxylic acid group..

Particularly preferred compounds of formula I include 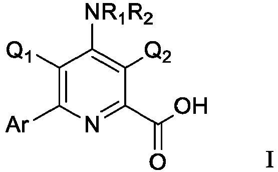 wherein
Q₁ represents H or F;
Q₂ represents Cl;
R₁ and R₂ represent H; and
Ar represents a polysubstituted aryl group 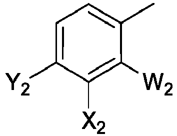 wherein
   W₂ represents F;
   X₂ represents C₁-C₄ alkoxy;
   Y₂ represents Cl;
   and agriculturally acceptable derivatives of the carboxylic acid group.
In some embodiments, X₂ is methoxy. Some of the compounds of formula I include e.g., 4-amino-3-chloro-6-(4-chloro-2-fluoro-3-methoxyphenyl)pyridine-2-carboxylic acid (halauxifen) and agriculturally acceptable salts and esters thereof, such as halauxifen-methyl, which is methyl 4-amino-3-chloro-6-(4-chloro-2-fluoro-3-methoxyphenyl)-2-pyridinecarboxylate

The term herbicide is used herein to mean an active ingredient that kills, controls or otherwise adversely modifies the growth of plants. As used herein, a herbicidally effective or vegetation controlling amount is an amount of active ingredient that causes a "herbicidal effect", *i.e.* an adversely modifying effect and includes deviations from natural development, killing, regulation, desiccation, and retardation.

As used herein, selective control of undesirable vegetation means preventing, reducing, killing, or otherwise adversely modifying the development of the undesirable vegetation at any stage of growth in a body of water.

As used herein, the terms "plants" and "vegetation" include, but are not limited to, germinant seeds, emerging seedlings, plants emerging from vegetative propagules, and established vegetation.

As used herein, immature vegetation refers to small vegetative plants prior to reproductive stage, and mature vegetation refers to vegetative plants during and after the reproductive stage.

As used herein, agriculturally acceptable salts and esters of the compound of formula I refer to salts and esters that (a) do not substantially affect the herbicidal activity and (b) are or can by hydrolyzed, oxidized, metabolized, or otherwise converted in plants or solid to the corresponding carboxylic acid which, depending on the pH, may be in the dissociated or undissociated form. Exemplary salts include those derived from alkali or alkaline earth metals and those derived from ammonia and amines. Exemplary cations include sodium, potassium, magnesium, and ammonium cations of the formula:

R¹R²R³R⁴N⁺

wherein R¹, R², R³ and R⁴ each, independently represents hydrogen or C₁-C₁₂ alkyl, C₃-C₁₂ alkenyl or C₃-C₁₂ alkynyl, each of which is optionally substituted by one or more hydroxy, C₁-C₄ alkoxy, C₁-C₄ alkylthio or phenyl groups, provided that R¹, R², R³ and R⁴ are sterically compatible. Additionally, any two of R¹, R², R³ and R⁴ together may represent an aliphatic difunctional moiety containing one to twelve carbon atoms and up to two oxygen or sulfur atoms. Salts can be prepared by treatment with a metal hydroxide, such as sodium hydroxide, with an amine, such as ammonia, trimethylamine, diethanolamine, 2-methylthiopropylamine, bisallylamine, 2-butoxyethylamine, morpholine, cyclododecylamine, or benzylamine or with a tetraalkylammonium hydroxide, such as tetramethylammonium hydroxide or choline hydroxide.

Exemplary esters include those derived from C₁-C₁₂ alkyl, C₃-C₁₂ alkenyl, C₃-C₁₂ alkynyl or C₇-C₁₀ aryl-substituted alkyl alcohols, such as methyl alcohol, isopropyl alcohol, 1-butanol, 2-ethylhexanol, butoxyethanol, methoxypropanol, allyl alcohol, propargyl alcohol, cyclohexanol or unsubstituted or substituted benzyl alcohols. Benzyl alcohols may be substituted with from 1-3 substituents independently selected from halogen, C₁-C₄ alkyl or C₁-C₄ alkoxy. Esters can be prepared by coupling of the acids with the alcohol using any number of suitable activating agents such as those used for peptide couplings such as dicyclohexylcarbodiimide (DCC) or carbonyl diimidazole (CDI); by reacting the acids with alkylating agents such as alkylhalides or alkylsulfonates in the presence of a base such as triethylamine or lithium carbonate; by reacting the corresponding acid chloride of an acid with an appropriate alcohol; by reacting the corresponding acid with an appropriate alcohol in the presence of an acid catalyst or by transesterification.

### II. Compositions

### A. Compounds

Herbicidal activity is exhibited by the compounds when they are applied to the body of water containing the plants to be controlled at any stage of growth. The effect observed depends upon the plant species to be controlled, the stage of growth of the plant, the application parameters of dilution, the particle size of solid components, the environmental conditions at the time of use, the specific compound employed, the specific adjuvants and carriers employed, and the like, as well as the amount of chemical applied. These and other factors can be adjusted to promote herbicidal action. In the present methods, the compositions described herein are applied to relatively immature and mature undesirable vegetation to achieve the maximum control of weeds.

In some embodiments, the compound of formula I or salt or ester thereof and complementary herbicides are applied at the same time, either as a combination formulation or as a tank-mix, or as a sequential application.

In the methods described herein, the compositions of formula I can be applied to a body of water to be treated by the use of conventional dusters, sprayers, and granule applicators, by addition to injection water, and by other conventional means known to those skilled in the art.

In some embodiments, the concentration of the active ingredients in the compositions described herein is from 0.0005 to 98 percent by weight. In some embodiments, the concentration is from 0.0006 to 90 percent by weight. In compositions designed to be employed as concentrates, the active ingredients, in certain embodiments, are present in a concentration from 0.1 to 98 weight percent, and in certain embodiments 0.5 to 90 weight percent. Such compositions are, in certain embodiments, diluted with an inert carrier, such as water, before application. The diluted compositions usually applied to weeds or the locus of weeds contain, in certain embodiments, 0.0006 to 3.0 weight percent active ingredient and in certain embodiments contain 0.01 to 0.3 weight percent.

In the methods described herein, the amount of the compound of formula I applied to a body of water to be treated can be applied in a concentrated form such that the final dilution level of the compound of formula I is between 1 parts-per-billion (ppb) and 2000 ppb. In additional embodiments of the methods described herein, the amount of the compound of formula I applied to a body of water to be treated can be applied in a concentrated form such that the final dilution level of the compound of formula I is between 1 ppb and 1500 ppb, 1 ppb and 1250 ppb, 1 ppb and 1000 ppb, 1 ppb and 900 ppb, 1 ppb and 800 ppb, 1 ppb and 700 ppb, 1 ppb and 600 ppb, 1 ppb and 500 ppb, 1 ppb and 450 ppb, 1 ppb and 400 ppb, 1 ppb and 375 ppb, 1 ppb and 350 ppb, 1 ppb and 325 ppb, 1 ppb and 300 ppb, 1 ppb and 275 ppb, 1 ppb and 250 ppb, 1 ppb and 225 ppb, 1 ppb and 200 ppb, 1 ppb and 190 ppb, 1 ppb and 180 ppb, 1 ppb and 170 ppb, 1 ppb and 160 ppb, 1 ppb and 150 ppb, 1 ppb and 140 ppb, 1 ppb and 130 ppb, 1 ppb and 120 ppb, 1 ppb and 110 ppb, 1 ppb and 100 ppb, 1 ppb and 90 ppb, 1 ppb and 80 ppb, 1 ppb and 70 ppb, 1 ppb and 60 ppb, 1 ppb and 50 ppb, 1 ppb and 40 ppb, 1 ppb and 30 ppb, 1 ppb and 20 ppb, 1 ppb and 10 ppb, 10 ppb and 200 ppb, 10 ppb and 150 ppb, 10 ppb and 100 ppb, 10 ppb and 90 ppb, 10 ppb and 80 ppb, 10 ppb and 70 ppb, 10 ppb and 60 ppb, 10 ppb and 50 ppb, 10 ppb and 40 ppb, 10 ppb and 30 ppb, 10 ppb and 20 ppb, 20 ppb and 200 ppb, 20 ppb and 150 ppb, 20 ppb and 100 ppb, 20 ppb and 90 ppb, 20 ppb and 80 ppb, 20 ppb and 70 ppb, 20 ppb and 60 ppb, 20 ppb and 50 ppb, 20 ppb and 40 ppb, 20 ppb and 30 ppb, 30 ppb and 200 ppb, 30 ppb and 150 ppb, 30 ppb and 100 ppb, 30 ppb and 90 ppb, 30 ppb and 80 ppb, 30 ppb and 70 ppb, 30 ppb and 60 ppb, 30 ppb and 50 ppb, 30 ppb and 40 ppb, 40 ppb and 200 ppb, 40 ppb and 150 ppb, 40 ppb and 100 ppb, 40 ppb and 90 ppb, 40 ppb and 80 ppb, 40 ppb and 70 ppb, 40 ppb and 60 ppb, 40 ppb and 50 ppb, 50 ppb and 200 ppb, 50 ppb and 150 ppb, 50 ppb and 100 ppb, 50 ppb and 90 ppb, 50 ppb and 80 ppb, 50 ppb and 70 ppb, or 50 ppb and 60 ppb. In further embodiments of the methods described herein, the amount of the compound of formula I applied to a body of water to be treated can be applied in a concentrated form such that the final dilution level of the compound of formula I is 0.1 ppb, 0.25 ppb, 0.5 ppb, 1 ppb, 2 ppb, 3 ppb, 4 ppb, 5 ppb, 6 ppb, 7 ppb, 8 ppb, 9 ppb, 10 ppb, 12 ppb, 14 ppb, 16 ppb, 18 ppb, 20 ppb, 22 ppb, 24 ppb, 26 ppb, 28 ppb, 30 ppb, 32 ppb, 34 ppb, 36 ppb, 38 ppb, 40 ppb, 42 ppb, 44 ppb, 46 ppb, 48 ppb, 50 ppb, 55 ppb, 60 ppb, 65 ppb, 70 ppb, 75 ppb, 80 ppb, 85 ppb, 90 ppb, 95 ppb, 100 ppb, 110 ppb, 120 ppb, 130 ppb, 140 ppb, 150 ppb, 160 ppb, 170 ppb, 180 ppb, 190 ppb, 200 ppb, 220 ppb, 240 ppb, 260 ppb, 280 ppb, 300 ppb, 320 ppb, 340 ppb, 360 ppb, 380 ppb, 400 ppb, 420 ppb, 440 ppb, 460 ppb, 480 ppb, of 500 ppb.

In other embodiments of the methods described herein, the amount of the compound of formula I applied to a body of water to be treated can be applied in a concentrated form such that the final dilution level of the compound of formula I is less than 5 ppb. In additional embodiments of the methods described herein, the amount of the compound of formula I applied to a body of water to be treated can be applied in a concentrated form such that the final dilution level of the compound of formula I is less than 6 ppb, less than 7 ppb, less than 8 ppb, less than 9 ppb, less than 10 ppb, less than 12 ppb, less than 14 ppb, less than 16 ppb, less than 18 ppb, less than 20 ppb, less than 22 ppb, less than 24 ppb, less than 26 ppb, less than 28 ppb, less than 30 ppb, less than 32 ppb, less than 34 ppb, less than 36 ppb, less than 38 ppb, less than 40 ppb, less than 42 ppb, less than 44 ppb, less than 46 ppb, less than 48 ppb, less than 50 ppb, less than 55 ppb, less than 60 ppb, less than 65 ppb, less than 70 ppb, less than 75 ppb, less than 80 ppb, less than 85 ppb, less than 90 ppb, less than 95 ppb, less than 100 ppb, less than 110 ppb, less than 120 ppb, less than 130 ppb, less than 140 ppb, less than 150 ppb, less than 160 ppb, less than 170 ppb, less than 180 ppb, less than 190 ppb, less than 200 ppb, less than 210 ppb, less than 220 ppb, less than 230 ppb, less than 240 ppb, less than 250 ppb, less than 275 ppb, less than 300 ppb, less than 325 ppb, less than 350 ppb, less than 400 ppb, less than 450 ppb, less than 500 ppb, less than 550 ppb, less than 600 ppb, less than 650 ppb, less than 700 ppb, less than 750 ppb, less than 800 ppb, less than 850 ppb, less than 900 ppb, less than 950 ppb, or less than 1000 ppb.

As described herein, the compounds of formula I are unexpectedly effective when applied to aquatic plants at very low concentrations, and/or can be used to improve selectivity for target aquatic weeds, thus having a lesser effect on non-target plant species due to reduced concentration of or exposure to the herbicidal agents.

### B. Other Actives

The mixtures described herein can be applied in conjunction with one or more other herbicides to control a wider variety of undesirable vegetation. When used in conjunction with other herbicides, the composition can be formulated with the other herbicide or herbicides, tank-mixed with the other herbicide or herbicides or applied sequentially with the other herbicide or herbicides. Some of the herbicides that can be employed in conjunction with the compositions and methods described herein include, but are not limited to acid, salt and ester forms of the following herbicides: 4-CPA, 4-CPB, 4-CPP, 3,4-DA, 2,4-D, 2,4-D choline salt, 2,4-DB, 3,4-DB, 3,4-DP, 2,3,6-TBA, 2,4,5-T, 2,4,5-TB, acetochlor, acifluorfen, aclonifen, acrolein, alachlor, allidochlor, alloxydim, allyl alcohol, alorac, ametridione, ametryn, amibuzin, amicarbazone, amidosulfuron, aminocyclopyrachlor, aminopyralid, amiprofos-methyl, amitrole, ammonium sulfamate, anilofos, anisuron, asulam, atraton, atrazine, azafenidin, azimsulfuron, aziprotryne, barban, BCPC, beflubutamid, benazolin, bencarbazone, benfluralin, benfuresate, bensulfuron-methyl, bensulide, bentazon, benthiocarb, benzadox, benzfendizone, benzipram, benzobicyclon, benzofenap, benzofluor, benzoylprop, benzthiazuron, bicyclopyrone, bifenox, bilanafos, bispyribac-sodium, borax, bromacil, bromobonil, bromobutide, bromofenoxim, bromoxynil, brompyrazon, butachlor, butafenacil, butamifos, butenachlor, buthidazole, buthiuron, butralin, butroxydim, buturon, butylate, cacodylic acid, cafenstrole, calcium chlorate, calcium cyanamide, cambendichlor, carbasulam, carbetamide, carboxazole, chlorprocarb, carfentrazone (e.g., carfentrazone-ethyl), CDEA, CEPC, chlomethoxyfen, chloramben, chloranocryl, chlorazifop, chlorazine, chlorbromuron, chlorbufam, chloreturon, chlorfenac, chlorfenprop, chlorflurazole, chlorflurenol, chloridazon, chlorimuron, chlornitrofen, chloropon, chlorotoluron, chloroxuron, chloroxynil, chlorpropham, chlorsulfuron, chlorthal, chlorthiamid, cinidon (e.g., cinidon-ethyl), cinmethylin, cinosulfuron, cisanilide, clethodim, cliodinate, clodinafop-propargyl, clofop, clomazone, clomeprop, cloprop, cloproxydim, clopyralid, cloransulam-methyl, CMA, copper sulfate, CPMF, CPPC, credazine, cresol, cumyluron, cyanatryn, cyanazine, cycloate, cyclopyrimorate, cyclosulfamuron, cycloxydim, cycluron, cyhalofop (e.g., cyhalofop-butyl), cyperquat, cyprazine, cyprazole, cypromid, daimuron, dalapon, dazomet, delachlor, desmedipham, desmetryn, di-allate, dicamba, dichlobenil, dichloralurea, dichlormate, dichlorprop, dichlorprop-P, diclofop-methyl, diclosulam, diethamquat, diethatyl, difenopenten, difenoxuron, difenzoquat, diflufenican, diflufenzopyr, dimefuron, dimepiperate, dimethachlor, dimethametryn, dimethenamid, dimethenamid-P, dimexano, dimidazon, dinitramine, dinofenate, dinoprop, dinosam, dinoseb, dinoterb, diphenamid, dipropetryn, diquat, disul, dithiopyr, diuron, DMPA, DNOC, DSMA, EBEP, eglinazine, endothal, epronaz, EPTC, erbon, esprocarb, ethbenzamide, ethalfluralin, ethametsulfuron, ethidimuron, ethiolate, ethobenzamid, etobenzamid, ethofumesate, ethoxyfen, ethoxysulfuron, etinofen, etnipromid, etobenzanid, EXD, fenasulam, fenoprop, fenoxaprop (e.g., fenoxaprop-P-ethyl), fenoxaprop-P-ethyl + isoxadifen-ethyl, fenoxasulfone, fenquinotrione, fenteracol, fenthiaprop, fentrazamide, fenuron, ferrous sulfate, flamprop, flamprop-M, flazasulfuron, florasulam, fluazifop (e.g., fluazifop-P-butyl), fluazolate, flucarbazone, flucetosulfuron, fluchloralin, flufenacet, flufenican, flufenpyr (e.g., flufenpyr-ethyl), flumetsulam, flumezin, flumiclorac (e.g., flumiclorac-pentyl), flumioxazin, flumipropyn, fluometuron, fluorodifen, fluoroglycofen, fluoromidine, fluoronitrofen, fluothiuron, flupoxam, flupropacil, flupropanate, flupyrsulfuron, fluridone, flurochloridone, fluroxypyr, flurtamone, fluthiacet, fomesafen, foramsulfuron, fosamine, fumiclorac, furyloxyfen, halauxifen, halosafen, halosulfuron (e.g., halosulfuron-methyl), haloxydine, haloxyfop-methyl, haloxyfop-P (e.g., haloxyfop-P-methyl), hexachloroacetone, hexaflurate, hexazinone, imazamethabenz, imazamox, imazapic, imazapyr, imazaquin, imazosulfuron, indanofan, indaziflam, iodobonil, iodomethane, iodosulfuron, iodosulfuron-ethyl-sodium, iofensulfuron, ioxynil, ipazine, ipfencarbazone, iprymidam, isocarbamid, isocil, isomethiozin, isonoruron, isopolinate, isopropalin, isoproturon, isouron, isoxaben, isoxachlortole, isoxaflutole, isoxapyrifop, karbutilate, ketospiradox, lactofen, lenacil, linuron, MAA, MAMA, MCPA esters and amines, MCPA-thioethyl, MCPB, mecoprop, mecoprop-P, medinoterb, mefenacet, mefluidide, mesoprazine, mesosulfuron, mesotrione, metam, metamifop, metamitron, metazachlor, metazosulfuron, metflurazon, methabenzthiazuron, methalpropalin, methazole, methiobencarb, methiozolin, methiuron, methometon, methoprotryne, methyl bromide, methyl isothiocyanate, methyldymron, metobenzuron, metobromuron, metolachlor, metosulam, metoxuron, metribuzin, metsulfuron, metsulfuron-methyl, molinate, monalide, monisouron, monochloroacetic acid, monolinuron, monuron, morfamquat, MSMA, naproanilide, napropamide, napropamide-M, naptalam, neburon, nicosulfuron, nipyraclofen, nitralin, nitrofen, nitrofluorfen, norflurazon, noruron, OCH, orbencarb, ortho-dichlorobenzene, orthosulfamuron, oryzalin, oxadiargyl, oxadiazon, oxapyrazon, oxasulfuron, oxaziclomefone, oxyfluorfen, paraflufen-ethyl, parafluron, paraquat, pebulate, pelargonic acid, pendimethalin, penoxsulam, pentachlorophenol, pentanochlor, pentoxazone, perfluidone, pethoxamid, phenisopham, phenmedipham (e.g., phenmedipham-ethyl), phenobenzuron, phenylmercury acetate, picloram, picolinafen, pinoxaden, piperophos, potassium arsenite, potassium azide, potassium cyanate, pretilachlor, primisulfuron (e.g., primisulfuron-methyl), procyazine, prodiamine, profluazol, profluralin, profoxydim, proglinazine, prohexadione-calcium, prometon, prometryn, propachlor, propanil, propaquizafop, propazine, propham, propisochlor, propoxycarbazone, propyrisulfuron, propyzamide, prosulfalin, prosulfocarb, prosulfuron, proxan, prynachlor, pydanon, pyraclonil, pyraflufen (e.g., pyraflufen-ethyl), pyrasulfotole, pyrazogyl, pyrazolynate, pyrazosulfuron-ethyl, pyrazoxyfen, pyribenzoxim, pyributicarb, pyriclor, pyridafol, pyridate, pyriftalid, pyriminobac, pyrimisulfan, pyrithiobac-sodium, pyroxasulfone, pyroxsulam, quinclorac, quinmerac, quinoclamine, quinonamid, quizalofop, quizalofop-P-ethyl, rhodethanil, rimsulfuron, saflufenacil, S-metolachlor, sebuthylazine, secbumeton, sethoxydim, siduron, simazine, simeton, simetryn, SMA, sodium arsenite, sodium azide, sodium chlorate, sulcotrione, sulfallate, sulfentrazone, sulfometuron, sulfosate, sulfosulfuron, sulfuric acid, sulglycapin, swep, TCA, tebutam, tebuthiuron, tefuryltrione, tembotrione, tepraloxydim, terbacil, terbucarb, terbuchlor, terbumeton, terbuthylazine, terbutryn, tetrafluron, thenylchlor, thiazafluron, thiazopyr, thidiazimin, thidiazuron, thiencarbazone-methyl, thifensulfuron, thifensulfuron-methyl, thiobencarb, tiafenacil, tiocarbazil, tioclorim, tolpyralate, topramezone, tralkoxydim, triafamone, tri-allate, triasulfuron, triaziflam, tribenuron (e.g., tribenuron-methyl), tricamba, triclopyr (e.g., triclopyr choline salt), triclopyr esters and salts, tridiphane, trietazine, trifloxysulfuron, trifludimoxazin, trifluralin, triflusulfuron, trifop, trifopsime, trihydroxytriazine, trimeturon, tripropindan, tritac tritosulfuron, vernolate, xylachlor, benzyl 4-amino-3-chloro-5-fluoro-6-(4-chloro-2-fluoro-3-methoxyphenyl)pyridine-2-carboxylate and salts, choline salts, esters, optically active isomers and mixtures thereof.

Exemplary additional pesticides include, but are not limited to, diquat dibromide, copper salts, endothal, flumioxazin, carfentrazone-ethyl, fluridone, topramezone, 2,4-D, 2,4-D choline salt, triclopyr, triclopyr choline salt, penoxsulam, imazamox, and bispyribac-sodium, including, with respect to the carboxylic acid containing pesticides, agriculturally acceptable salts or esters thereof.

### C. Safeners

In some embodiments, the compositions described herein are employed in combination with one or more herbicide safeners, such as AD-67 (MON 4660), benoxacor, benthiocarb, brassinolide, cloquintocet (mexyl), cyometrinil, daimuron, dichlormid, dicyclonon, dimepiperate, disulfoton, fenchlorazole-ethyl, fenclorim, flurazole, fluxofenim, furilazole, harpin proteins, isoxadifen-ethyl, jiecaowan, jiecaoxi, mefenpyr-diethyl, mephenate, naphthalic anhydride (NA), oxabetrinil, R29148 and *N*-phenyl-sulfonylbenzoic acid amides, to enhance their selectivity.

### D. Adjuvants/Carriers

In some embodiments, compositions provided herein further comprise at least one or more agriculturally acceptable adjuvant or carrier. Suitable adjuvants or carriers should not be phytotoxic to valuable plant species, particularly at the concentrations employed in applying the compositions for selective aquatic weed control, and should not react chemically with herbicidal components or other composition ingredients. Such mixtures can be designed for application directly to weeds or their locus or can be concentrates or formulations that are normally diluted with additional carriers and adjuvants before application. They can be solids, such as, for example, dusts, granules, water-dispersible granules, or wettable powders, or liquids, such as, for example, emulsifiable concentrates, solutions, emulsions or suspensions. They can also be provided as a pre-mix or tank-mixed.

Suitable agricultural adjuvants and carriers include, but are not limited to, crop oil concentrate; nonylphenol ethoxylate; benzylcocoalkyldimethyl quaternary ammonium salt; blend of petroleum hydrocarbon, alkyl esters, organic acid, and anionic surfactant; C₉-C₁₁ alkylpolyglycoside; phosphated alcohol ethoxylate; natural primary alcohol (C₁₂-C₁₆) ethoxylate; di-sec-butylphenol EO-PO block copolymer; polysiloxane-methyl cap; nonylphenol ethoxylate + urea ammonium nitrate; emulsified methylated seed oil; tridecyl alcohol (synthetic) ethoxylate (8EO); tallow amine ethoxylate (15 EO); PEG(400) dioleate-99; paraffinic oil, alkoxylated alcohol non-ionic surfactant; mineral oil, surfactant blend.

Liquid carriers that can be employed include water and organic solvents. The organic solvents include, but are not limited to, petroleum fractions or hydrocarbons such as mineral oil, aromatic solvents, paraffinic oils, and the like; vegetable oils such as soybean oil, rapeseed oil, olive oil, castor oil, sunflower seed oil, coconut oil, corn oil, cottonseed oil, linseed oil, palm oil, peanut oil, safflower oil, sesame oil, tung oil and the like; esters of the above vegetable oils; esters of monoalcohols or dihydric, trihydric, or other lower polyalcohols (4-6 hydroxy containing), such as 2-ethyl hexyl stearate, *n*-butyl oleate, isopropyl myristate, propylene glycol dioleate, di-octyl succinate, di-butyl adipate, di-octyl phthalate and the like; esters of mono, di and polycarboxylic acids and the like. Specific organic solvents include, but are not limited to toluene, xylene, petroleum naphtha, crop oil, acetone, methyl ethyl ketone, cyclohexanone, trichloroethylene, perchloroethylene, ethyl acetate, amyl acetate, butyl acetate, propylene glycol monomethyl ether and diethylene glycol monomethyl ether, methyl alcohol, ethyl alcohol, isopropyl alcohol, amyl alcohol, ethylene glycol, propylene glycol, glycerine, *N*-methyl-2-pyrrolidinone, *N,N*-dimethyl alkylamides, dimethyl sulfoxide, liquid fertilizers and the like. In certain embodiments, water is the carrier for the dilution of concentrates.

Suitable solid carriers include but are not limited to talc, pyrophyllite clay, silica, attapulgus clay, kaolin clay, kieselguhr, chalk, diatomaceous earth, lime, calcium carbonate, bentonite clay, Fuller's earth, cottonseed hulls, wheat flour, soybean flour, pumice, wood flour, walnut shell flour, lignin, cellulose, and the like.

In some embodiments, the compositions described herein further comprise one or more surface-active agents. In some embodiments, such surface-active agents are employed in both solid and liquid compositions, and in certain embodiments those designed to be diluted with carrier before application. The surface-active agents can be anionic, cationic or nonionic in character and can be employed as emulsifying agents, wetting agents, suspending agents, or for other purposes. Surfactants which may also be used in the present formulations are described, *inter alia,* in McCutcheon's Detergents and Emulsifiers Annual, MC Publishing Corporation: Ridgewood, NJ, 1998 and in Encyclopedia of Surfactants, Vol. I-III, Chemical Publishing Company: New York, 1980-81. Surface-active agents include, but are not limited to salts of alkyl sulfates, such as diethanolammonium lauryl sulfate; alkylarylsulfonate salts, such as calcium dodecylbenzenesulfonate; alkylphenol-alkylene oxide addition products, such as nonylphenol-C₁₈ ethoxylate; alcohol-alkylene oxide addition products, such as tridecyl alcohol-C₁₆ ethoxylate; soaps, such as sodium stearate; alkylnaphthalene-sulfonate salts, such as sodium dibutylnaphthalenesulfonate; dialkyl esters of sulfosuccinate salts, such as sodium di(2-ethylhexyl) sulfosuccinate; sorbitol esters, such as sorbitol oleate; quaternary amines, such as lauryl trimethylammonium chloride; polyethylene glycol esters of fatty acids, such as polyethylene glycol stearate; block copolymers of ethylene oxide and propylene oxide; salts of mono and dialkyl phosphate esters; vegetable or seed oils such as soybean oil, rapeseed/canola oil, olive oil, castor oil, sunflower seed oil, coconut oil, corn oil, cottonseed oil, linseed oil, palm oil, peanut oil, safflower oil, sesame oil, tung oil and the like; and esters of the above vegetable oils, and in certain embodiments, methyl esters.

In some embodiments, these materials, such as vegetable or seed oils and their esters, can be used interchangeably as an agricultural adjuvant, as a liquid carrier or as a surface active agent.

Other exemplary additives for use in the compositions provided herein include but are not limited to compatibilizing agents, antifoam agents, sequestering agents, neutralizing agents and buffers, corrosion inhibitors, dyes, odorants, spreading agents, penetration aids, sticking agents, dispersing agents, thickening agents, freezing point depressants, antimicrobial agents, and the like. The compositions may also contain other compatible components, for example, other herbicides, plant growth regulants, fungicides, insecticides, and the like and can be formulated with liquid fertilizers or solid, particulate fertilizer carriers such as ammonium nitrate, urea and the like.

### III. Methods of use

Methods of controlling aquatic weeds in a body of water, including providing in the body of water a composition containing a herbicidally effective amount of a compound of formula I 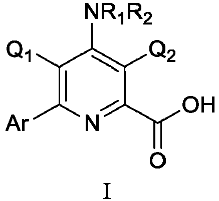 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group selected from the group consisting of
   a) 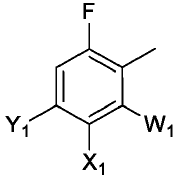 wherein
      W₁ represents halogen;
      X₁ represents F, Cl, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -CN, -NR₃R₄ or fluorinated acetyl or propionyl;
      Y₁ represents C₁-C₄ alkyl, C₁-C₄ haloalkyl, halogen or -CN, or, when X₁ and Y₁ are taken together, represents -O(CH₂)ₙO- wherein n = 1 or 2; and
      R₃ and R₄ independently represent H or C₁-C₄ alkyl;
   b) 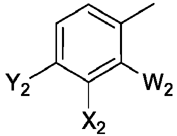 wherein
      W₂ represents F or Cl;
      X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
      Y₂ represents halogen, C₁-C₄ alkyl, C₁-C₄ haloalkyl or -CN, or, when W₂ represents F, X₂ and Y₂ taken together represent -O(CH₂)ₙO- wherein n = 1 or 2; and
      R₃ and R₄ independently represent H or C₁-C₆ alkyl; and
   c) 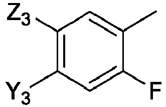 wherein
      Y₃ represents halogen, -CN or -CF₃;
      Z₃ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl; and
      R₃ and R₄ independently represent H, or C₁-C₆ alkyl;
   and agriculturally acceptable derivatives of the carboxylic acid group,
   wherein the aquatic weeds are selected from submersed aquatic plants.

Preferred compounds of formula I include 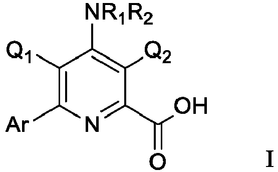 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group 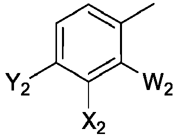 wherein
   W₂ represents F or Cl;
   X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -NR₃R₄ or fluorinated acetyl or propionyl;
   Y₂ represents halogen, C₁-C₄ alkyl, C₁-C₄ haloalkyl or -CN, or, when W₂ represents F, X₂ and Y₂ taken together represent -O(CH₂)ₙO- wherein n = 1 or 2; and R₃ and R₄ independently represent H or C₁-C₆ alkyl;
and agriculturally acceptable derivatives of the carboxylic acid group.

Particularly preferred compounds of formula I include 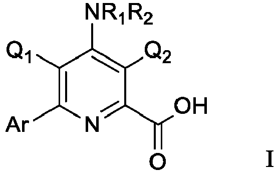 wherein
Q₁ represents H or F;
Q₂ represents Cl;
R₁ and R₂ represent H; and
Ar represents a polysubstituted aryl group 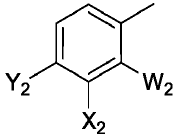 wherein
   W₂ represents F;
   X₂ represents C₁-C₄ alkoxy;
   Y₂ represents Cl;
   and agriculturally acceptable derivatives of the carboxylic acid group, are described herein. In some embodiments, X₂ is methoxy. In certain embodiments, the methods employ the compositions described herein.

The methods and compositions of the invention may be used in the complete or partial control of many noxious submersed aquatic plants and shoreline grasses. For example, the submersed plants include but are not limited to bladderwart (*Utricularia* spp.), baby's tears *(Micranthemum* spp.), common coontail (*Ceratophyllum demersum),* common elodea *(Elodea canadensis),* Brazilian elodea *(Egeria densa),* fanwort *(Cabomba caroliniana),* hydrilla (*Hydrilla verticillata),* naiad (*Najas* spp.), pondweed (*Potamogeton* spp.) and more specifically curlyleaf pondweed (*Potamogeton crispus)* and Illinois pondweed (*P*. *illinoensis*), horned pondweed (*Zannichellia palustris*), bacopa (*Bacopa* spp.), watermilfoil (*Myriophyllum* spp.) including Eurasian watermilfoil (*M spicatum*), tapegrass or American eelgrass (*Vallisneria americana*), and variable leaf watermilfoil (*Myriophyllum heterophyllum*); and the shoreline grasses barnyardgrass (*Echinochloa crus-galli*), and southern watergrass (*Hydrochloa caroliniensis*). Particularly preferred plant types for control in accordance with the invention include hydrilla, Eurasian watermilfoil and curlyleaf pondweed.

Bodies of water to be treated with the inventive methods will typically be fresh water bodies such as ponds, lakes, wet lands, reservoirs, rivers, streams, ditches or irrigation canals, although other bodies of water may also be treated in accordance with the invention. In certain embodiments bodies of water do not include rice paddies or rice fields.

In some embodiments, methyl 4-amino-3-chloro-6-(4-chloro-2-fluoro-3-methoxyphenyl)pyridine-2-carboxylate (halauxifen-methyl) and benzyl 4-amino-3-chloro-5-fluoro-6-(4-chloro-2-fluoro-3-methoxyphenyl)pyridine-2-carboxylate may be used to control herbicide resistant or tolerant weeds. In other embodiments, the methods employing the combination of methyl 4-amino-3-chloro-6-(4-chloro-2-fluoro-3-methoxyphenyl)pyridine-2-carboxylate (halauxifen-methyl) or benzyl 4-amino-3-chloro-5-fluoro-6-(4-chloro-2-fluoro-3-methoxyphenyl)pyridine-2-carboxylate and the compositions described herein may also be employed to control herbicide resistant or tolerant weeds. Exemplary resistant or tolerant weeds include, but are not limited to, biotypes resistant or tolerant to acetolactate synthase (ALS) or acetohydroxy acid synthase (AHAS) inhibitors (e.g., imidazolinones, sulfonylureas, pyrimidinylthiobenzoates, dimethoxy-pyrimidines, triazolopyrimidine sulfonamides, sulfonylaminocarbonyltriazolinones), photosystem II inhibitors (e.g., phenylcarbamates, pyridazinones, triazines, triazinones, uracils, amides, ureas, benzothiadiazinones, nitriles, phenylpyridazines), acetyl CoA carboxylase (ACCase) inhibitors (e.g., aryloxyphenoxypropionates, cyclohexanediones, phenylpyrazolines), synthetic auxins (e.g., benzoic acids, phenoxycarboxylic acids, pyridine carboxylic acids, quinoline carboxylic acids), auxin transport inhibitors (*e.g*., phthalamates, semicarbazones), photosystem I inhibitors (*e.g.*, bipyridyliums), 5-enolpyruvylshikimate-3-phosphate (EPSP) synthase inhibitors (*e.g.,* glyphosate), glutamine synthetase inhibitors (*e.g.,* glufosinate, bialafos), microtubule assembly inhibitors (*e.g*., benzamides, benzoic acids, dinitroanilines, phosphoramidates, pyridines), mitosis inhibitors (*e.g.*, carbamates), very long chain fatty acid (VLCFA) inhibitors (*e.g.*, acetamides, chloroacetamides, oxyacetamides, tetrazolinones), fatty acid and lipid synthesis inhibitors (*e.g.*, phosphorodithioates, thiocarbamates, benzofuranes, chlorocarbonic acids), protoporphyrinogen oxidase (PPO) inhibitors (*e.g.*, diphenylethers, *N*-phenylphthalimides, oxadiazoles, oxazolidinediones, phenylpyrazoles, pyrimidinediones, thiadiazoles, triazolinones), carotenoid biosynthesis inhibitors (*e.g.*, clomazone, amitrole, aclonifen, fluridone), phytoene desaturase (PDS) inhibitors (*e.g.*, amides, anilidex, furanones, phenoxybutan-amides, pyridiazinones, pyridines), 4-hydroxyphenyl-pyruvate-dioxygenase (HPPD) inhibitors (*e.g.*, callistemones, isoxazoles, pyrazoles, triketones), cellulose biosynthesis inhibitors (*e.g.*, nitriles, benzamides, quinclorac, triazolocarboxamides), herbicides with multiple modes-of-action such as quinclorac, and unclassified herbicides such as arylaminopropionic acids, difenzoquat, endothall, and organoarsenicals. Exemplary resistant or tolerant weeds include, but are not limited to, biotypes with resistance or tolerance to single or multiple herbicides, biotypes with resistance or tolerance to single or multiple chemical classes, biotypes with resistance or tolerance to single or multiple herbicide modes-of-action, and biotypes with single or multiple resistance or tolerance mechanisms (*e.g.*, target site resistance or metabolic resistance).

The compounds of formula I utilized should remain at herbicidally effective levels in the body of water in contact with the targeted plant to achieve control. Thus, in accordance with preferred methods of the invention, the herbicidal agents will be maintained in the treatment area or body of water under treatment for about 1 to 4 weeks, and in preferred embodiments for at least about four weeks, and typically in the range of about four to sixteen weeks or more. The concentration of the herbicidal agent may be maintained, when necessary, with the target plant to ensure efficacy, for example, through the use of sequential or bump treatments, or continuous injection, using the same agent. The described embodiments and following examples are for illustrative purposes.

### Examples

### Example 1. Control of Hydrilla

Apical sections (12 to 15 centimeters (cm) in length) of hydrilla (HYLLI) were planted into small pots (13.5 cm length x 3.75 cm diameter) containing topsoil amended with 14-14-14 slow release Osmocote® fertilizer (∼2.5 grams (g) Osmocote per kilogram (kg) soil). Approximately 5 to 7 cm of the apical section extended above the sediment at planting, and a sand cap was placed over the potting soil (∼2 cm deep). Plants were then transferred to 12 liter (L) acrylic tanks filled with well water. Tanks were maintained in a growth room with a 14:10 hour (h) photoperiod at 26 °C (±2 °C). Plants were allowed to grow for 17 days (d) before the treatments were initiated in triple replicate. Plants were treated with Compound 1 (halauxifen-methyl) at 0, 10, 100, 1000 parts per billion (ppb) with three replicates each. Treatments were only applied once. Plants were harvested at 33 d after initial treatment. At harvest, plants were rinsed free of algae and placed in paper sacks in a drying oven for 2 d at 70 °C temperature. Mean dry weights were determined and means separated using least significant differences (Table 1).

**Table 1. Final Aboveground Dry Weights of Hydrilla (HYLLI) at Thirty-three Days Following Submersed Exposure to Three Different Concentrations of Compound 1.**

| Test Material | Test Rate (ppb) | Dry Weight (grams)*of HYLLI |
|---|---|---|
| Compound 1 | 10 | 0.02 b |
| Compound 1 | 100 | 0.003 ab |
| Compound 1 | 1000 | 0 b |
| Untreated | 0 | 1.047 a |
| LSD (P=0.05) | | 0.3266 |
| Std Deviation | | 0.1795 |
| CV | | 83.9 |

| | | |
|---|---|---|
| *Means followed by the same letter are not significantly different (P = 0.5, LSD) | | |

Apical sections of hydrilla (HYLLI) were planted in potting soil amended with Osmocote® fertilizer in small pots (13.5 cm length x 3.75 cm diameter). A 2.54 cm sand layer was placed over the top of the potting soil. The pots containing hydrilla were placed into 14 L acrylic cylinders filled with well water and allowed to establish slight growth prior to treatment. Cylinders were then treated with Compound 1 (halauxifen-methyl 96 grams acid equivalent per liter (g ae/L) Suspension Concentrate (SC)) or Compound 2 (benzyl 4-amino-3-chloro-5-fluoro-6-(4-chloro-2-fluoro-3-methoxyphenyl)pyridine-2-carboxylate (125 grams active ingredient per liter (g ai/L) SC)) at 50, 100 and 200 parts per billion (ppb). All treatments were replicated 4 times. After 25 d of exposure, plants were harvested and weights were measured and compared to an untreated control treatment (Table 2).

**Table 2. Final Aboveground Dry Weights of Hydrilla (HYLLI) at Four Weeks Following 24-Hour Exposure to Three Different Concentrations of Compounds 1 and 2.**

| Test Material | Test Rate (ppb) | Dry Weight (grams)*of HYLLI |
|---|---|---|
| Compound 1 | 50 | 0.02 b |
| Compound 1 | 100 | 0.64 ab |
| Compound 1 | 200 | 0 b |
| Compound 2 | 50 | 0 b |
| Compound 2 | 100 | 0 b |
| Compound 2 | 200 | 0 b |
| Untreated | 0 | 2.48 a |

| | | |
|---|---|---|
| *Letters indicate statistically equivalent treatments (individual t tests N = 4 for Non-treated Reference an N = 3 for Treatments | | |

### Example 2. Control of Eurasian Watermilfoil and Curlyleaf Pondweed

Apical sections (12 to 15 cm in length) of Eurasian watermilfoil (MYPSP) and curlyleaf pondweed (PTMCR) were planted into small pots (13.5 cm length x 3.75 cm diameter) containing topsoil amended with 14-14-14 slow release Osmocote® fertilizer (∼2.5 g Osmocote/kg soil). Approximately 5 to 7 cm of the apical section extended above the sediment at planting, and a sand cap was placed over the potting soil (∼2 cm deep). Plants were then transferred to 12 L acrylic tanks filled with well water. Plants were allowed to grow for 7 d before the treatment was initiated in triple replicate. Plants were treated with Compound 1 (halauxifen-methyl 96 g ae/L SC) at 50, 100, 500 and 1000 ppb. Plants were harvested at 40 d. At harvest, plants were rinsed free of algae, roots and shoots were separated and placed in paper sacks in a drying oven for 4 days at 70 °C temperature, and dry weights were measured and compared to an untreated control treatment (Table 3).

**Table 3. Aboveground Dry Weights of Eurasian Watermilfoil (MYPSP) and Curlyleaf Pondweed (PTMCR) at Thirty Days Following a 24-Hour Exposure to Compound 1.**

| Test Material | Test Rate (ppb) | Dry Weight (grams)*of MYPSP | Dry Weight (grams)*of PTMCR |
|---|---|---|---|
| Compound 1 | 5 | 0.000 b | 0.117 a |
| Compound 1 | 10 | 0.000 b | 0.043 ab |
| Compound 1 | 50 | 0.000 b | 0.000 b |
| Compound 1 | 100 | 0.000 b | 0.000 b |
| Untreated | 0 | 1.257 a | 0.087 a |
| LSD (P=0.05) | | 0.1191 | 0.0818 |
| Std Deviation | | 0.0655 | 0.0449 |
| CV | | 26.05 | 91.1 |

| | | | |
|---|---|---|---|
| *Means followed by the same letter are not significantly different (P = 0.5, LSD) Hydrilla = *Hydrilla verticillata* (L.f.) Royle (HYLLI) Eurasian Watermilfoil = *Myriophyllum spicatum* L. (MYPSP) Curlyleaf Pondweed = *Potamogeton crispus* L. (PTMCR) Compound 1 = methyl 4-amino-3-chloro-6-(4-chloro-2-fluoro-3-methoxyphenyl)pyridine-2-carboxylate (halauxifen-methyl) Compound 2 = benzyl 4-amino-3-chloro-5-fluoro-6-(4-chloro-2-fluoro-3-methoxyphenyl)pyridine-2-carboxylate ppb = parts per billion | | | |

## Claims

1. A method for controlling aquatic weeds in a body of water which comprises providing in the body of water a composition comprising a herbicidally effective amount of a compound of formula I 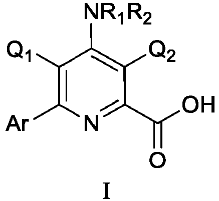 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group selected from the group consisting of
a) 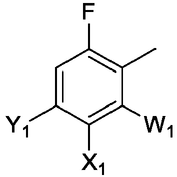 wherein
W₁ represents halogen;
X₁ represents F, Cl, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, -CN, -NR₃R₄ or fluorinated acetyl or propionyl;
Y₁ represents C₁-C₄ alkyl, C₁-C₄ haloalkyl, halogen or-CN, or, when X₁ and Y₁ are taken together, represents -O(CH₂)ₙO- wherein n = 1 or 2; and
R₃ and R₄ independently represent H or C₁-C₄ alkyl;
b) 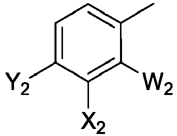 wherein
W₂ represents F or Cl;
X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, - NR₃R₄ or fluorinated acetyl or propionyl;
Y₂ represents halogen, C₁-C₄ alkyl, C₁-C₄ haloalkyl or-CN, or, when W₂ represents F, X₂ and Y₂ taken together represent -O(CH₂)ₙO- wherein n = 1 or 2; and
R₃ and R₄ independently represent H or C₁-C₆ alkyl; and
c) 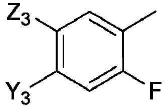 wherein
Y₃ represents halogen, -CN or -CF₃;
Z₃ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, - NR₃R₄ or fluorinated acetyl or propionyl; and
R₃ and R₄ independently represent H, or C₁-C₆ alkyl;
and agriculturally acceptable derivatives of the carboxylic acid group,
wherein the aquatic weeds are selected from submersed aquatic plants.

2. The method of claim 1, wherein the compound of formula I 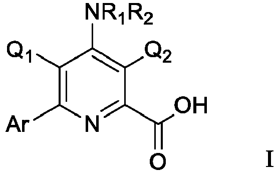 wherein
Q₁ represents H or F;
Q₂ represents a halogen with the proviso that when Q₁ is H then Q₂ is Cl or Br;
R₁ and R₂ independently represent H, C₁-C₆ alkyl, C₃-C₆ alkenyl, C₃-C₆ alkynyl, hydroxy, C₁-C₆ alkoxy, amino, C₁-C₆ acyl, C₁-C₆ carboalkoxy, C₁-C₆ alkylcarbamyl, C₁-C₆ alkylsulfonyl, C₁-C₆ trialkylsilyl or C₁-C₆ dialkyl phosphonyl or R₁ and R₂ taken together with N represent a 5- or 6-membered saturated ring; and
Ar represents a polysubstituted aryl group 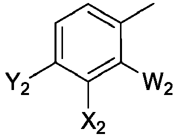 wherein
W₂ represents F or Cl;
X₂ represents F, Cl, -CN, -NO₂, C₁-C₄ alkyl, C₁-C₄ alkoxy, C₁-C₄ alkylthio, C₁-C₄ alkylsulfinyl, C₁-C₄ alkylsulfonyl, C₁-C₄ haloalkyl, C₁-C₄ haloalkoxy, C₁-C₄ alkoxy-substituted C₁-C₄ alkyl, C₁-C₄ alkoxy-substituted C₁-C₄ alkoxy, - NR₃R₄ or fluorinated acetyl or propionyl;
Y₂ represents halogen, C₁-C₄ alkyl, C₁-C₄ haloalkyl or-CN, or, when W₂ represents F, X₂ and Y₂ taken together represent -O(CH₂)ₙO- wherein n = 1 or 2; and
R₃ and R₄ independently represent H or C₁-C₆ alkyl;
and agriculturally acceptable derivatives of the carboxylic acid group.

3. The method of claim 1, wherein the compound of formula I 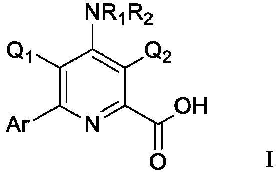 wherein
Q₁ represents H or F;
Q₂ represents Cl;
R₁ and R₂ represent H; and
Ar represents a polysubstituted aryl group 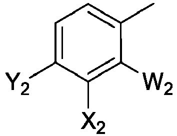 wherein
W2 represents F;
X₂ represents C₁-C₄ alkoxy;
Y₂ represents Cl;
and agriculturally acceptable derivatives of the carboxylic acid group.

4. The method of claim 1, wherein the aquatic weeds include hydrilla, curlyleaf pondweed or Eurasian watermilfoil.

5. The method of any of claims 1-4, wherein the compound of formula I is methyl 4-amino-3-chloro-6-(4-chloro-2-fluoro-3-methoxyphenyl)pyridine-2-carboxylate (halauxifen-methyl).

6. The method of any of claims 1-4, wherein the compound of formula I is benzyl 4-amino-3-chloro-5-fluoro-6-(4-chloro-2-fluoro-3-methoxyphenyl)pyridine-2-carboxylate.

7. The method of any of claims 1-6, wherein the final dilution level of the compound of formula I in the body of water is from about 5 parts-per-billion (ppb) to about 2000 ppb, preferably from about 5 parts-per-billion (ppb) to about 200 ppb.

8. The method of any of claims 1-6, wherein the final dilution level of the compound of formula I in the body of water is less than about 1000 parts-per-billion (ppb), preferably less than about 200 ppb.

9. The method of any of claims 1-8, wherein the compound of formula I is applied in conjunction with one or more other herbicides to control a wider variety of undesirable vegetation.

10. The method of claim 9, wherein the additional herbicide includes one or more herbicides selected from the group consisting of diquat dibromide, copper salts, endothal, flumioxazin, carfentrazone-ethyl, fluridone, topramezone, 2,4-D, 2,4-D choline salt, triclopyr, triclopyr choline salt, penoxsulam, imazamox, bispyribac-sodium, and agriculturally acceptable salts or esters thereof.

11. The method of any of claims 1-10, wherein the aquatic weeds comprise a herbicide resistant or tolerant weed.

12. The method of claim 11, wherein the resistant or tolerant weed is a biotype with resistance or tolerance to single or multiple herbicides or single or multiple chemical classes, or inhibitors of single or multiple herbicide modes-of-action.

13. The method of claim 11 or claim 12, wherein the resistant or tolerant weed is a biotype resistant or tolerant to acetolactate synthase (ALS) or acetohydroxy acid synthase (AHAS) inhibitors, photosystem II inhibitors, acetyl CoA carboxylase (ACCase) inhibitors, photosystem I inhibitors, 5-enolpyruvyl-shikimate-3-phosphate (EPSP) synthase inhibitors, microtubule assembly inhibitors, lipid synthesis inhibitors, protoporphyrinogen oxidase (PPO) inhibitors, carotenoid biosynthesis inhibitors, bleachers, very long chain fatty acid (VLCFA) inhibitors, phytoene desaturase (PDS) inhibitors, glutamine synthetase inhibitors, 4-hydroxyphenyl-pyruvate-dioxygenase (HPPD) inhibitors, mitosis inhibitors, cellulose biosynthesis inhibitors, herbicides with multiple modes-of-action, quinclorac, arylaminopropionic acids, difenzoquat, endothall or organoarsenicals.

## Patentansprüche

1. Ein Verfahren zur Bekämpfung von Wasserunkräutern in einem Gewässer, welches das Bereitstellen einer Zusammensetzung umfassend eine herbizidwirksame Menge einer Verbindung der Formel I 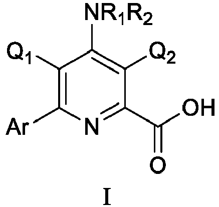 wobei
Q₁ H oder F darstellt;
Q₂ ein Halogen darstellt, unter der Voraussetzung, dass, wenn Q₁ H ist, Q₂ dann Cl oder Br ist;
R₁ und R₂ unabhängig H, C₁-C₆-Alkyl, C₃-C₆-Alkenyl, C₃-C₆-Alkynyl, Hydroxy, C₁-C₆-Alkoxy, Amino, C₁-C₆-Acyl, C₁-C₆-Carboalkoxy, C₁-C₆-Alkylcarbamyl, C₁-C₆-Alkylsulfonyl, C₁-C₆-Trialkylsilyl oder C₁-C₆-Dialkylphosphonyl darstellen oder R₁ und R₂ zusammen genommen mit N einen 5- oder 6-gliedrigen gesättigten Ring darstellen; und
Ar eine mehrfach substituierte Arylgruppe ausgewählt aus der Gruppe bestehend aus
a) 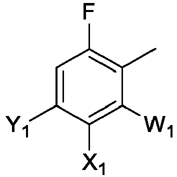 wobei
W₁ Halogen darstellt;
X₁ F, Cl, C₁-C₄-Alkyl, C₁-C₄-Alkoxy, C₁-C₄-Alkylthio, C1-C₄-Alkylsulfinyl, C₁-C₄-Alkylsulfonyl, C₁-C₄-Haloalkyl, C₁-C₄-Haloalkoxy, C₁-C₄-alkoxysubstituiertes C₁-C₄-Alkyl, C₁-C₄-alkoxysubstituiertes C₁-C₄-Alkoxy, - CN, -NR₃R₄ oder fluoriertes Acetyl oder Propionyl darstellt;
Y1 C1-C4-Alkyl, C1-C4-Haloalkyl, Halogen oder -CN darstellt, oder, wenn X1 und Y1 zusammengenommen werden,-O(CH2) nO- darstellt, wobei n = 1 oder 2 ist; und
R₃ und R₄ unabhängig H oder C₁-C₄-Alkyl darstellen;
b) 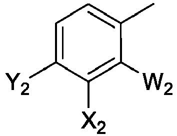 wobei
W₂ F oder Cl darstellt;
X2 F, Cl, -CN, -NO₂, C1-C4-Alkyl, C1-C4-Alkoxy, C1-C4-Alkylthio, C1-C4-Alkylsulfinyl, C1-C4-Alkylsulfonyl, C1-C4-Haloalkyl, C1-C4-Haloalkoxy, C1-C4-alkoxysubstituiertes C1-C4-Alkyl, C1-C4-alkoxysubstituiertes C1-C4-Alkoxy, -NR3R4 oder fluoriertes Acetyl oder Propionyl darstellt;
Y2 Halogen, C1-C4-Alkyl, C1-C4-Haloalkyl oder-CN, oder, wenn W2 F darstellt, X2 and Y2 zusammengenommen -O(CH2) nOdarstellen, wobei n = 1 oder 2 ist; und
R₃ und R₄ unabhängig H oder C₁-C₆-Alkyl darstellen; und
c) 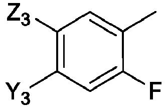 wobei
Y3 Halogen, -CN oder-CF3 darstellt;
Z3 F, Cl, -CN, -NO₂, C1-C4-Alkyl, C1-C4-Alkoxy, C1-C4-Alkylthio, C1-C4-Alkylsulfinyl, C1-C4-Alkylsulfonyl, C1-C4-Haloalkyl, C1-C4-Haloalkoxy, C1-C4-alkoxysubstituiertes C1-C4-Alkyl, C1-C4-alkoxysubstituiertes C1-C4-Alkoxy, -NR3R4 oder fluoriertes Acetyl oder Propionyl darstellt; und
R3 und R4 unabhängig H oder C1-C6-Alkyl darstellen;
und landwirtschaftlich akzeptable Derivate der Carbonsäuregruppe,
in dem Gewässer umfasst,
wobei die Wasserunkräuter aus submersen Wasserpflanzen ausgewählt sind.

2. Das Verfahren gemäß Anspruch 1, wobei die Verbindung der Formel I 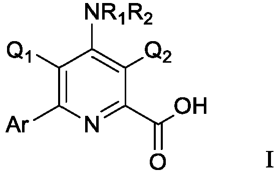 wobei
Q₁ H oder F darstellt;
Q₂ ein Halogen darstellt, unter der Voraussetzung, dass, wenn Q₁ ist, H Q₂ dann Cl oder Br ist;
R₁ und R₂ unabhängig H, C₁-C₆-Alkyl, C₃-C₆-Alkenyl, C₃-C₆-Alkynyl, Hydroxy, C₁-C₆-Alkoxy, Amino, C₁-C₆-Acyl, C₁-C₆-Carboalkoxy, C₁-C₆-Alkylcarbamyl, C₁-C₆-Alkylsulfonyl, C₁-C₆-Trialkylsilyl oder C₁-C₆-Dialkylphosphonyl darstellen oder R₁ und R₂ zusammen genommen mit N einen 5- oder 6-gliedrigen gesättigten Ring darstellen; und
Ar eine mehrfach substituierte Arylgruppe darstellt, 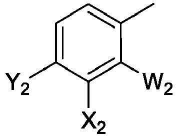 wobei
W₂ F oder Cl darstellt;
X₂ F, Cl, -CN, -NO₂, C₁-C₄-Alkyl, C₁-C₄-Alkoxy, C₁-C₄-Alkylthio, C₁-C₄-Alkylsulfinyl, C₁-C₄-Alkylsulfonyl, C₁-C₄-Haloalkyl, C₁-C₄-Haloalkoxy, C₁-C₄-alkoxysubstituiertes C₁-C₄-Alkyl, C₁-C₄-alkoxysubstituiertes C₁-C₄-Alkoxy, -NR₃R₄ oder fluoriertes Acetyl oder Propionyl darstellt;
Y₂ Halogen, C₁-C₄-Alkyl, C₁-C₄-Haloalkyl oder-CN, oder, wenn W₂ F darstellt, X₂ und Y₂ zusammengenommen -O(CH₂)ₙO-darstellen, wobei n = 1 oder 2 ist; und
R₃ und R₄ unabhängig H oder C₁-C₆-Alkyl darstellen;
und landwirtschaftlich akzeptable Derivate der Carbonsäuregruppe.

3. Das Verfahren gemäß Anspruch 1, wobei die Verbindung der Formel I 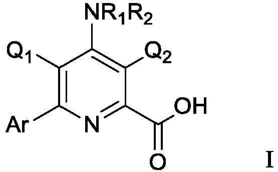 wobei
Q₁ H oder F darstellt;
Q₂ Cl darstellt;
R₁ und R₂ H darstellen; und
Ar eine mehrfach substituierte Arylgruppe darstellt, 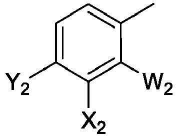 wobei
W₂ F darstellt;
X₂ C₁-C₄-Alkoxy darstellt;
Y₂ Cl darstellt;
und landwirtschaftlich akzeptable Derivate der Carbonsäuregruppe.

4. Das Verfahren gemäß Anspruch 1, wobei das Wasserunkraut Grundnässe, krauses Laichkraut oder Ähriges Tausendblatt umfasst.

5. Das Verfahren gemäß irgendeinem der Ansprüche 1-4, wobei die Verbindung der Formel I 4-amino-3-chlor-6-(4-chlor-2-fluor-3-methoxyphenyl)pyridin-2-Carbonsäuremethylester (Halauxifen-Methyl) ist.

6. Das Verfahren gemäß irgendeinem der Ansprüche 1-4, wobei die Verbindung der Formel I 4-amino-3-chlor-5-fluor-6-(4-chlor-2-fluor-3-methoxyphenyl)pyridin-2-Carbonsäurebenzyl ist.

7. Das Verfahren gemäß irgendeinem der Ansprüche 1-6, wobei der endgültige Verdünnungsgrad der Verbindung der Formel I in dem Gewässer von etwa 5 Teilen pro Milliarde (ppb) bis etwa 2000 ppb beträgt, vorzugsweise von etwa 5 Teilen pro Milliarde (ppb) bis etwa 200 ppb.

8. Das Verfahren gemäß irgendeinem der Ansprüche 1-6, wobei der endgültige Verdünnungsgrad der Verbindung der Formel I in dem Gewässer weniger als etwa 1000 Teile pro Milliarde (ppb) beträgt, vorzugsweise weniger als etwa 200 ppb.

9. Das Verfahren gemäß irgendeinem der Ansprüche 1-8, wobei die Verbindung der Formel I zusammen mit einem oder mehreren weiteren Herbiziden angewandt wird, um eine größere Vielzahl unerwünschter Vegetation zu bekämpfen.

10. Das Verfahren gemäß Anspruch 9, wobei das zusätzliche Herbizid ein oder mehrere Herbizide ausgewählt aus der Gruppe bestehend aus Diquatdibromid, Kupfersalzen, Endothal, Flumioxazin, Carfentrazon-Ethyl, Fluridon, Topramezon, 2,4-D, 2,4-D Cholinsalz, Triclopyr, Triclopyrcholinsalz, Penoxsulam, Imazamox, Bispyribac-Natrium und landwirtschaftlich akzeptablen Salzen oder Estern davon beinhaltet.

11. Das Verfahren gemäß irgendeinem der Ansprüche 1 - 10, wobei die Wasserunkräuter ein herbizidresistentes oder -tolerantes Unkraut umfassen.

12. Das Verfahren gemäß Anspruch 11, wobei das resistente oder tolerante Unkraut ein Biotyp mit Resistenz oder Toleranz gegenüber einem oder mehreren Herbiziden oder einer oder mehreren chemischen Klassen oder Hemmern einer oder mehrerer herbizider Wirkweisen ist.

13. Das Verfahren gemäß Anspruch 11 oder Anspruch 12, wobei das resistente oder tolerante Unkraut ein Biotyp ist, der resistent oder tolerant gegenüber Acetolactatsynthase-(ALS)- oder Acetohydroxysäuresynthase-(AHAS)-Hemmern, Photosystem-II-Hemmern, Acetyl-CoA-Carboxylase-(ACCase)-Hemmern, Photosystem-I-Hemmern, 5-Enolpyruvylshikimat-3-phosphat-(EPSP)-Synthase-Hemmern, Hemmern der Mikrotubulianordnung, Lipidsynthese-Hemmern, Protoporphyrinogen-Oxidase-(PPO)-Hemmern, Hemmern der Carotinoid-Biosynthese, Hemmern sehr langkettiger Fettsäuren (VLCFA), Phytoen-Desaturase-(PDS)-Hemmern, Glutamin-Synthetase-Hemmern, 4-Hydroxyphenylpyruvat-Dioxygenase-(HPPD)-Hemmern, Mitose-Hemmern, Hemmern der Cellulosebiosynthese, Herbiziden mit mehreren Wirkweisen, Quinclorac, Arylaminopropionsäuren, Difenzoquat, Endothal oder Organoarsenverbindungen ist.

## Revendications

1. Procédé de lutte contre des mauvaises herbes aquatiques dans une étendue d'eau, qui comporte le fait d'introduire dans l'étendue d'eau une composition comprenant, en une quantité à effet herbicide, un composé de formule I 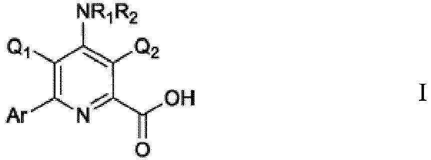 dans laquelle
- Q₁ représente un atome d'hydrogène ou de fluor,
- Q₂ représente un atome d'halogène, sous réserve que, si Q₁ représente un atome d'hydrogène, alors Q₂ représente un atome de chlore ou de brome,
- les symboles R₁ et R₂ représentent chacun, indépendamment, un atome d'hydrogène ou un groupe alkyle en C₁-C₆, alcényle en C₃-C₆, alcynyle en C₃-C₆, hydroxyle, alcoxy en C₁-C₆, amino, acyle en C₁-C₆, alcoxycarbonyle en C₁-C₆, alkylcarbamyle en C₁-C₆, alkylsulfonyle en C₁-C₆, tri(alkyle en C₁-C₆)silyle, ou di(alkyle en C₁-C₆)phosphonyle, ou bien R₁ et R₂ représentent des entités formant, conjointement avec l'atome d'azote, un cycle saturé comportant 5 ou 6 chaînons,
- et Ar représente un groupe aryle porteur de plusieurs substituants, choisi dans l'ensemble formé par les suivants :
a) les groupes de formule 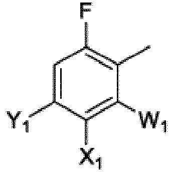 dans laquelle
W₁ représente un atome d'halogène,
X₁ représente un atome de fluor ou de chlore, un groupe alkyle en C₁-C₄, alcoxy en C₁-C₄, alkylthio en C₁-C₄, alkyl-sulfinyle en C₁-C₄, alkyl-sulfonyle en C₁-C₄, halogéno-alkyle en C₁-C₄, halogéno-alcoxy en C₁-C₄, alkyle en C₁-C₄ à substituant alcoxy en C₁-C₄, alcoxy en C₁-C₄ à substituant alcoxy en C₁-C₄, cyano, acétyle fluoré ou propionyle fluoré, ou un groupe symbolisé par -NR₃R₄,
et Y₁ représente un atome d'halogène ou un groupe cyano, alkyle en C₁-C₄ ou halogéno-alkyle en C₁-C₄,
ou bien X₁ et Y₁ représentent des entités formant conjointement un groupe symbolisé par -O(CH₂)ₙO- où l'indice n vaut 1 ou 2, étant entendu que R₃ et R₄ représentent chacun, indépendamment, un atome d'hydrogène ou un groupe alkyle en C₁-C₄,
b) les groupes de formule 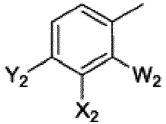 dans laquelle
W₂ représente un atome de fluor ou de chlore,
X₂ représente un atome de fluor ou de chlore, un groupe cyano, nitro, alkyle en C₁-C₄, alcoxy en C₁-C₄, alkylthio en C₁-C₄, alkyl-sulfinyle en C₁-C₄, alkyl-sulfonyle en C₁-C₄, halogéno-alkyle en C₁-C₄, halogéno-alcoxy en C₁-C₄, alkyle en C₁-C₄ à substituant alcoxy en C₁-C₄, alcoxy en C₁-C₄ à substituant alcoxy en C₁-C₄, acétyle fluoré ou propionyle fluoré, ou un groupe symbolisé par -NR₃R₄,
et Y₂ représente un atome d'halogène ou un groupe cyano, alkyle en C₁-C₄ ou halogéno-alkyle en C₁-C₄,
ou bien, si W₂ représente un atome de fluor, X₂ et Y₂ représentent des entités formant conjointement un groupe symbolisé par -O(CH₂)ₙO- où l'indice n vaut 1 ou 2,
étant entendu que R₃ et R₄ représentent chacun, indépendamment, un atome d'hydrogène ou un groupe alkyle en C₁-C₆,
c) et les groupes de formule 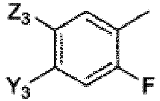 dans laquelle
Y₃ représente un atome d'halogène ou un groupe cyano ou trifluorométhyle,
et Z₃ représente un atome de fluor ou de chlore, un groupe cyano, nitro, alkyle en C₁-C₄, alcoxy en C₁-C₄, alkylthio en C₁-C₄, alkyl-sulfinyle en C₁-C₄, alkyl-sulfonyle en C₁-C₄, halogéno-alkyle en C₁-C₄, halogéno-alcoxy en C₁-C₄, alkyle en C₁-C₄ à substituant alcoxy en C₁-C₄, alcoxy en C₁-C₄ à substituant alcoxy en C₁-C₄, acétyle fluoré ou propionyle fluoré, ou un groupe symbolisé par -NR₃R₄,
étant entendu que R₃ et R₄ représentent chacun, indépendamment, un atome d'hydrogène ou un groupe alkyle en C₁-C₄,
ou un dérivé d'un tel composé au niveau de son groupe acide carboxylique, admissible en agriculture,
étant entendu que les mauvaises herbes aquatiques sont choisies parmi les végétaux aquatiques submergés.

2. Procédé conforme à la revendication 1, dans lequel le composé est un composé de formule I 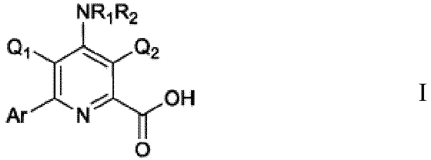 dans laquelle
- Q₁ représente un atome d'hydrogène ou de fluor,
- Q₂ représente un atome d'halogène, sous réserve que, si Q₁ représente un atome d'hydrogène, alors Q₂ représente un atome de chlore ou de brome,
- les symboles R₁ et R₂ représentent chacun, indépendamment, un atome d'hydrogène ou un groupe alkyle en C₁-C₆, alcényle en C₃-C₆, alcynyle en C₃-C₆, hydroxyle, alcoxy en C₁-C₆, amino, acyle en C₁-C₆, alcoxycarbonyle en C₁-C₆, alkylcarbamyle en C₁-C₆, alkylsulfonyle en C₁-C₆, tri(alkyle en C₁-C₆)silyle, ou di(alkyle en C₁-C₆)phosphonyle, ou bien R₁ et R₂ représentent des entités formant, conjointement avec l'atome d'azote, un cycle saturé comportant 5 ou 6 chaînons,
- et Ar représente un groupe aryle porteur de plusieurs substituants, de formule 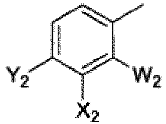 dans laquelle
W₂ représente un atome de fluor ou de chlore,
X₂ représente un atome de fluor ou de chlore, un groupe cyano, nitro, alkyle en C₁-C₄, alcoxy en C₁-C₄, alkylthio en C₁-C₄, alkyl-sulfinyle en C₁-C₄, alkyl-sulfonyle en C₁-C₄, halogéno-alkyle en C₁-C₄, halogéno-alcoxy en C₁-C₄, alkyle en C₁-C₄ à substituant alcoxy en C₁-C₄, alcoxy en C₁-C₄ à substituant alcoxy en C₁-C₄, acétyle fluoré ou propionyle fluoré, ou un groupe symbolisé par -NR₃R₄, et Y₂ représente un atome d'halogène ou un groupe cyano, alkyle en C₁-C₄ ou halogéno-alkyle en C₁-C₄,
ou bien, si W₂ représente un atome de fluor, X₂ et Y₂ représentent des entités formant conjointement un groupe symbolisé par -O(CH₂)ₙO- où l'indice n vaut 1 ou 2,
étant entendu que R₃ et R₄ représentent chacun, indépendamment, un atome d'hydrogène ou un groupe alkyle en C₁-C₆,
ou un dérivé d'un tel composé au niveau de son groupe acide carboxylique, admissible en agriculture.

3. Procédé conforme à la revendication 1, dans lequel le composé est un composé de formule I 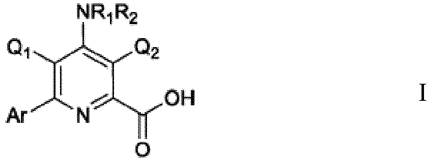 dans laquelle
- Q₁ représente un atome d'hydrogène ou de fluor,
- Q₂ représente un atome de chlore,
- les symboles R₁ et R₂ représentent chacun un atome d'hydrogène,
- et Ar représente un groupe aryle porteur de plusieurs substituants, de formule 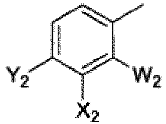 dans laquelle W₂ représente un atome de fluor, X₂ représente un groupe alcoxy en C₁-C₄, et Y₂ représente un atome de chlore,
ou un dérivé d'un tel composé au niveau de son groupe acide carboxylique, admissible en agriculture.

4. Procédé conforme à la revendication 1, dans lequel les mauvaises herbes aquatiques comprennent l'hydrille, le potamot crêpu et le myriophylle à épis.

5. Procédé conforme à l'une des revendications 1 à 4, dans lequel le composé de formule I est du 4-amino-3-chloro-6-(4-chloro-2-fluoro-3-méthoxy-phényl)-pyridine-2-carboxylate de méthyle (halauxifène-méthyl).

6. Procédé conforme à l'une des revendications 1 à 4, dans lequel le composé de formule I est du 4-amino-3-chloro-5-fluoro-6-(4-chloro-2-fluoro-3-méthoxy-phényl)-pyridine-2-carboxylate de benzyle.

7. Procédé conforme à l'une des revendications 1 à 6, dans lequel le niveau final de dilution du composé de formule I dans l'étendue d'eau vaut d'environ 5 ppmd (parties par milliard) à environ 2000 ppmd, et de préférence, d'environ 5 ppmd à environ 200 ppmd.

8. Procédé conforme à l'une des revendications 1 à 6, dans lequel le niveau final de dilution du composé de formule I dans l'étendue d'eau vaut moins d'environ 1000 ppmd (parties par milliard), et de préférence, moins d'environ 200 ppmd.

9. Procédé conforme à l'une des revendications 1 à 8, dans lequel on applique le composé de formule I conjointement avec un ou plusieurs autre(s) herbicide(s), afin de lutter contre un plus large spectre de végétaux indésirables.

10. Procédé conforme à la revendication 9, dans lequel l'herbicide supplémentaire inclut un ou plusieurs herbicide(s) choisi(s) dans l'ensemble formé par les suivants : dibromure de diquat, sels de cuivre, endothal, flumioxazine, carfentrazone-éthyl, fluridone, topramézone, 2,4-D, sel de choline de 2,4-D, triclopyr, sel de choline de triclopyr, pénoxsulame, imazamox, bispyribac-sodium, et leurs esters et sels admissibles en agriculture.

11. Procédé conforme à l'une des revendications 1 à 10, dans lequel les mauvaises herbes aquatiques comprennent une mauvaise herbe résistante ou tolérante.

12. Procédé conforme à la revendication 11, dans lequel la mauvaise herbe résistante ou tolérante est un biotype doté d'une résistance ou d'une tolérance vis-à-vis d'un seul ou de plusieurs herbicide(s) ou d'une seule ou de plusieurs classe(s) de composés chimiques, ou d'inhibiteurs dotés d'un seul ou de plusieurs mode(s) d'action d'herbicide.

13. Procédé conforme à la revendication 11 ou 12, dans lequel la mauvaise herbe résistante ou tolérante est un biotype résistant ou tolérant vis-à-vis d'inhibiteurs de l'acéto-lactate synthétase (ALS) ou de l'acéto-hydroxyacide synthétase (AHAS), d'inhibiteurs du photosystème II, d'inhibiteurs de l'acétyl-CoA carboxylase (ACCase), d'inhibiteurs du photosystème I, d'inhibiteurs de la 5-énol-pyruvyl-shikimate-3-phosphate synthétase (EPSP), d'inhibiteurs de l'assemblage des microtubules, d'inhibiteurs de la synthèse de lipides, d'inhibiteurs de la protoporphyrinogène oxydase (PPO), d'inhibiteurs de la biosynthèse de caroténoïdes, d'agents de dépigmentation, d'inhibiteurs des acides gras à très longue chaîne (VLCFA ou AGTLC), d'inhibiteurs de la phytoène désaturase (PDS), d'inhibiteurs de la glutamine synthétase, d'inhibiteurs de la 4-hydroxyphényl-pyruvate dioxygénase (HPPD), d'inhibiteurs de la mitose, d'inhibiteurs de la biosynthèse de cellulose, d'herbicides dotés de plusieurs modes d'action, du quinclorac, des acides arylamino-propioniques, du difenzoquat, de l'endothal ou des organo-arséniés.
